# EUROPÄISCHE PATENTANMELDUNG

(11) **EP 2 196 138 A2**
(43) Veröffentlichungstag der Anmeldung: **16.06.2010**
(21) Anmeldenummer: 09014974.1
(22) Anmeldetag: 03.12.2009
(51) Int. Cl.: A61B 5/00, A61B 5/0205, A61B 5/0402, A61B 5/08

(54) **System und Verfahren zum Stresstraining eines Benutzers**

(30) Priorität: 12.12.2008 DE 102008061997
(71) Anmelder: Karlsruher Institut für Technologie Institut für Technologie der Informationsverarbeitung, 76131 Karlsruhe (DE)
(72) Erfinder: Stork, Wilhelm, 76831 Impflingen (DE); Großmann, Ulrich, 76133 Karlsruhe (DE); Hey, Stefan, 76831 Impflingen (DE); Ottenbacher, Jörg, 76131 Karlsruhe (DE); Stumpp, Jürgen, 76337 Waldbronn (DE)

(57) **Zusammenfassung**

Die Erfindung sieht ein System zum Stresstraining eines Benutzers mit einer Sensorvorrichtung, eine Sensoreinrichtung, eine Signalverarbeitungseinrichtung und mindestens einer Signaleinheit vor, bei dem die Signalverarbeitungseinrichtung zur Bestimmung eines Werts für den Stress des Benutzers aus mindestens einem mit der psychophysiologischen Aktivierung des Benutzers korrelierten Parameter ausgebildet ist und, dass die Signaleinheit zur Aufgabe einer Rückmeldung an den Benutzer bei Über- bzw. Unterschreiten von definierten Schwellwerten der Aktivierung ausgebildet ist. Weiter beinhaltet die Erfindung ein Verfahren zum Stresstraining eines Benutzers, welches dadurch gekennzeichnet ist, dass die Signalverarbeitungseinrichtung zur Bestimmung eines Werts für den Stress des Benutzers aus mindestens einem mit der psychophysiologischen Aktivierung des Benutzers korrelierten Parameter ausgebildet ist und, dass die Signaleinheit zur Aufgabe einer Rückmeldung an den Benutzer bei Über- bzw. Unterschreiten von definierten Schwellwerten der Aktivierung aus einem vorgegeben Zeitprofil durch den Benutzer ausgebildet ist.

## Beschreibung

Die Erfindung betrifft ein System zum Stresstraining eines Benutzers mit einer Sensoreinrichtung, einer Signalverarbeitungseinrichtung und mindestens einer Signalisierungseinheit sowie ein entsprechendes Verfahren.

Die Variable Stress wird heute in zweierlei Weise verstanden, zum einen als Disstress der Reize, die ein Individuum als negativ, überfordernd, bedrohlich etc. empfindet, und als Eustress in Form von Reizen, die als positiv interpretiert bzw. erlebt werden. Die psychophysiologische Aktivierung eines Individuums kann in verschiedenen Bereichen liegen, neben einem optimalen, ggf. auch situationsabhängig optimalen Zustand ist eine Hyperaktivierung möglich. Demgegenüber ist eine Hyperdeaktivierung zu unterscheiden, die eine Relaxion, Schlaf und Erschöpfung beinhaltet und in Ruhezeiten als optimal angesehen werden kann, nicht aber in Zeiten, in denen aktive Tätigkeit erforderlich ist und in diesen als negativ angesehen wird.

Die Erfindung zielt auf die Bestimmung der psychophysiologischen Aktivierung durch Messung physiologischer Parameter ab, die allerdings Auswirkungen beinhalten, die auf eine subjektive Bewertung zurückgehen.

Die WO 2006/113900 A2 zeigt ein in der Hand tragbares Biofeedbackgerät, mittels dessen RSA-Wellen (Wellen der respiratorische Sinusarrhythmie) erfasst werden. Der Hochpünkt der Herzfrequenz beim Einatmen, der sogenannte RSA-Druckpunkt wird an den Benutzer zurückgegeben, so dass dieser seine Atmung entsprechend anpassen kann. Dies soll zur Aktivierung des parasympathischen Nervensystems führen und eine Entspannungsreaktion auslösen, damit hierdurch eine Stresssituation unterbrochen wird. Nachteilig ist, dass zur Durchführung des Verfahrens die Aufmerksamkeit des Benutzers erforderlich ist. Der Stress als solcher wird nicht gemessen und erkannt, damit kann dem Benutzer kein Hinweis auf die Unterbrechung von Stresssituationen gegeben werden. Nachteilig ist weiterhin, dass das Gerät, dadurch dass es in der Hand getragen werden muss, den Benutzer beeinträchtigt.

Die WO 2006/082565 A1 betrifft die Anordnung von Sensoren zur Erfassung physiologischer Parameter, die in Textilien eingearbeitet sind. Mit diesem Gerät kann der Stresslevel einer Person bestimmt und ein entsprechendes Feedback zurückgegeben werden. Eine Aussage über die Signalverarbeitung und die Bestimmung des Stresslevels selbst ist nicht offenbart.

Die DE 2002 2905 U1 beschreibt eine miniaturisierte Sensorvorrichtung zur Überwachung der Disstress-Eustress-Relaxionsfunktion (des Wohlbefindens) einer Person, welche nach dem Prinzip des Biofeedbacks arbeitet und mittels Sensoren kontinuierlich Vitalparameter misst. Die Sensorvorrichtung kann bevorzugt in mit der Haut in Kontakt stehende Gegenstände eingebaut sein, z.B. einer Armbanduhr, und informiert den Benutzer über dessen Stresszustand. Es wird keine Aussage über die Signalverarbeitung gemacht.

Der Erfindung liegt die Aufgabe zugrunde, ein System und ein Verfahren zum Stresstraining eines Benutzers zu schaffen, mittels derer ein Benutzer einen psychophysiologischen Aktivierungsgrad in einem optimalen Bereich erreichen kann.

Erfindungsgemäß wird die genannte Aufgabe mit einem System der eingangs genannten Art gelöst, welches dadurch gekennzeichnet ist, dass die Signalverarbeitungseinrichtung zur Bestimmung eines Werts für den Stress des Benutzers aus mindestens einem mit der psychophysiologischen Aktivierung des Benutzers korrelierten Parameter ausgebildet ist und, dass die Signalisierungseinheit zur Aufgabe einer Rückmeldung an den Benutzer bei Über- bzw. Unterschreiten von definierten Schwellwerten der Aktivierung ausgebildet ist.

Entsprechend sieht ein erfindungsgemäßes Verfahren zur Lösung der genannten Aufgabe vor, dass die Signalverarbeitungseinrichtung zur Bestimmung eines Werts für den Stress des Benutzers aus mindestens einem mit der psychophysiologischen Aktivierung des Benutzers korrelierten Parameter ausgebildet ist und, dass die Signalisierungseinheit zur Aufgabe einer Rückmeldung an den Benutzer bei Über- bzw. Unterschreiten von definierten Schwellwerten der Aktivierung ausgebildet ist.

Bevorzugte Weiterbildungen des erfindungsgemäßen Verfahrens sehen dabei vor, dass aus gemessenen Sensordaten Merkmalsparameter extrahiert werden, hieraus Parameterdaten erzeugt werden, aufgrund derer mindestens ein Ausgabeparameter bestimmt wird, wobei insbesondere die Extraktion der Merkmalsparameter, die Berechnung der Parameterdaten die Berechnung der Ausgabeparameter mehrstufig erfolgt.

In Weiterbildung ist vorgesehen, dass in die Bestimmung eines Merkmalsparameters mindestens zwei Sensordaten, in die Bestimmung von Parameterdaten mindesten zwei Merkmalsparameter und/oder in die Bestimmung von Ausgabeparametern mindestens zwei Parameterdaten eingehen, als Sensordaten z.B. EKG, Atmung und/oder Beschleunigung des Benutzers gemessen werden und dass aus den Sensordaten als Merkmalsparameter z.B. Herzrate, Atemfrequenz, Energieumsatz, Aktivitätslevel, RR-Abstände (zeitlicher Abstande der R-Zacken im EKG), Aktivitätsklassen, Muskeltonus und phasische und tonische Werte der elektrodermalen Aktivität (EDA) extrahiert werden.

Darüber hinaus kann erfindungsgemäß vorgesehen sein, dass als Parameterdaten mindestens einen mit der psychophysiologischen Aktivierung korrelierender Parameter aus Herzratenvariabilität (HRV), respiratorischer Sinusarrhythmie (RSA) und/oder additional heart rate (AHR) bestimmt werden.

Eine bevorzugte Ausgestaltung des erfindungsgemäßen Systems sieht vor, dass die Sensoreinrichtung in an sich bekannter Weise am Körper tragbar ist, und insbesondere als Brustgurt, Gürtel, Top oder als Armbanduhr ausgestaltet sein kann.

Hierbei kann dann in weiterer Ausgestaltung des Systems vorgesehen sein, dass die Sensoreinrichtung automatisch kontextbezogen an- und ausschaltbar ist, insbesondere durch An- oder Ablegen in Form von Schließen des Brustgurtes, durch Körperwärme, bei An- bzw. Abwesenheit von Vitalparametern, durch Überwachung der Potentialdifferenz zweier Elektroden, durch Anschalten bei Bewegung und Abschalten bei vollständiger Ruhe.

Entsprechend der genannten zu erfassenden Sensordaten kann die Sensoreinrichtung innerhalb des Systems verschiedene Sensoren aufweisen, so kann insbesondere vorgesehen sein, dass die Sensoren der Sensoreinrichtung in mindestens zwei als EKG- (Elektrokardiogramm), EMG- (Elektromyogramm), EDA-, Atmungs-, Puls-, Aktivitäts-, Bewegungs-, Lage-, Temperatur- und/oder Feuchtigkeitssensor ausgebildet sind.

Weitere bevorzugte Ausgestaltungen des erfindungsgemäßen Systems sehen vor, dass die Sensoreinrichtung drahtlos mit der Signalverarbeitungseinrichtung verbunden ist, wobei insbesondere die Signalverarbeitungseinrichtungen als Smartphone, PC und/oder Uhr ausgestaltet sind und weiterhin mindestens eine der Signalverarbeitungseinrichtungen zumindest zeitweise drahtlos oder drahtverbunden über das Internet mit einer Webanwendung verbunden ist, womit die Sensoreinrichtung selbst zumindest zeitweise direkt über eine drahtlose Signalverbindung mit dem Internet verbindbar ist und damit Kontextinformationen aus externen Datenquellen beziehbar sind.

Die Signalisierungseinheit kann im Rahmen des Systems ebenfalls in unterschiedlicher Weise ausgebildet sein, so als optisch anzeigende Signalisierungseinheit, wobei aber bevorzugte Ausgestaltungen vorsehen, dass die Signalisierungseinheit, welche eine Rückmeldung an den Benutzer gibt, taktil als Vibrationsalarm und/oder als akustische Signalisierungseinheit ausgebildet ist.

Um gemessene Sensordaten und hieraus berechnete Merkmalsparameter, Parameterdaten und/oder Ausgabeparameter mit subjektiven Empfindungen des Patienten korrelieren zu können, sieht eine Weiterbildung des erfindungsgemäßen Systems vor, dass die Sensoreinrichtung eine Eingabeeinrichtung (z.B. Taster) aufweist, mittels dessen Messdaten Markierungen für Ereignisse zuordenbar sind und, dass die Eingabeeinrichtung Eingaben mittels eines Beschleunigungssensor durch Antippen des Systems zulässt.

Zur Durchführung der verfahrensmäßigen Verarbeitung und dieser entsprechend ist das Sensorsystem weiterhin dahingehend ausgebildet, dass die Sensoreinrichtung zur Bestimmung mindestens eines Wertes für chronischen Stress und die Balance verschiedener Aktivierungsbereiche eines Benutzers, (Ausgewogenheit zwischen Über- und Unteraktivierung bezüglich Dauer und Intensität) ausgebildet ist, wobei insbesondere ein interner Datenspeicher der Sensoreinrichtung zum Speichern erfasster Sensordaten und/oder daraus berechneten weiteren Parametern und das Sensorsystem mit Hilfe einer Signalverbindung die gespeicherten Daten, ebenso aktuelle Sensordaten, Merkmalsparameter, Parameterdaten und/oder Ausgabeparameter in Echtzeit, bestimmten Zeitintervallen und/oder bei Verfügbarkeit der Signalverbindung an die Signalverarbeitungseinrichtung überträgt.

Neben einer momentanen Ausgabe von Signalen bei Überschreiten oder Unterschreiten von einem optimalen psychophysiologischen Aktivitätsbereich begrenzenden Schwellwerten sieht die Erfindung in weiterer Ausgestaltung vor, dass die Sensoreinrichtung konfigurierbar ist, indem insbesondere persönliche Daten, ein Zeitprofil der gewünschten Zeitabschnitte für Aktivierung bzw. Situationsunterbrechung einer Stresssituation und persönliche Aktivierungswerte bzw. Auslöseverzögerungen einstellbar sind. Darüber hinaus kann vorgesehen sein, dass die Optionen Aktivierung und Situationsunterbrechung einer Stresssituation manuell aktivierbar bzw. deaktivierbar sind, wobei insbesondere die Sensoreinrichtung individuell kalibrierbar ist, indem insbesondere einerseits Kalibrierungswerte aufgrund von Eigenschaften des Benutzers aus einer Datenbank ermittelt werden, andererseits während einer bestimmten Tragedauer das Verhalten des Benutzers analysiert, d.h. explizit Ruhe- bzw. Schlafphasen erkannt und aus dem Vergleich zu aktiven Phasen des Benutzers eine Grundlinie für einzelne Merkmalsparameter und Parameterdaten errechnet wird.

Weitere Vorteile und Merkmale der Erfindung ergeben sich aus den Ansprüchen und sind aus der nachfolgenden Beschreibung in den Ausführungsbeispielen der Entwicklung unter Bezug auf die Zeichnungen im Einzelnen erläutert.

Die erfindungsgemäße Vorrichtung mit dem erfindungsgemäßen Verfahren wird nachstehend anhand begleitender Zeichnungen in einer bevorzugten Ausführungsform beschrieben. Es zeigt:
- Fig. 1: eine schematische Darstellung der Komponenten der erfindungsgemäßen Vorrichtung;
- Fig. 2: die Komponenten der Sensoreinrichtung in einer schematischen Übersicht in der Interaktion mit dem Benutzer;
- Fig. 3: ein Ausführungsbeispiel des erfindungsgemäßen Verfahrens zur Signalverarbeitung in Form des mehrstufigen Diagramms;
- Fig. 4: ein Ablaufdiagramm des erfindungsgemäßen Verfahrens;
- Fig. 5: ein Ablaufdiagramm der Kalibrierung der erfindungsgemäßen Vorrichtung.

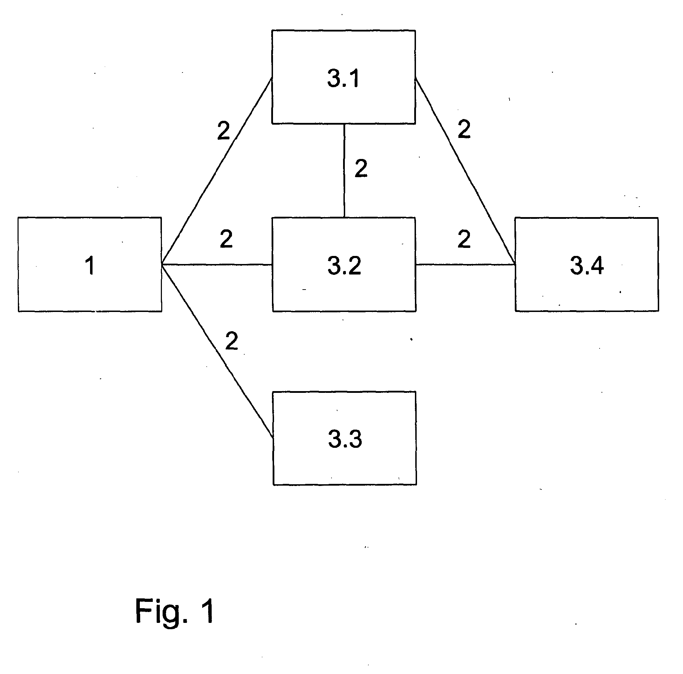

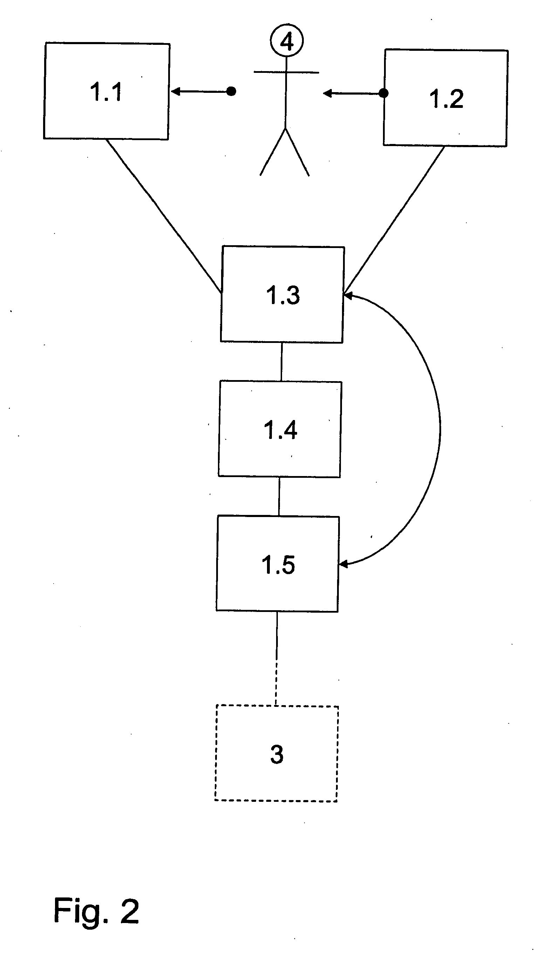

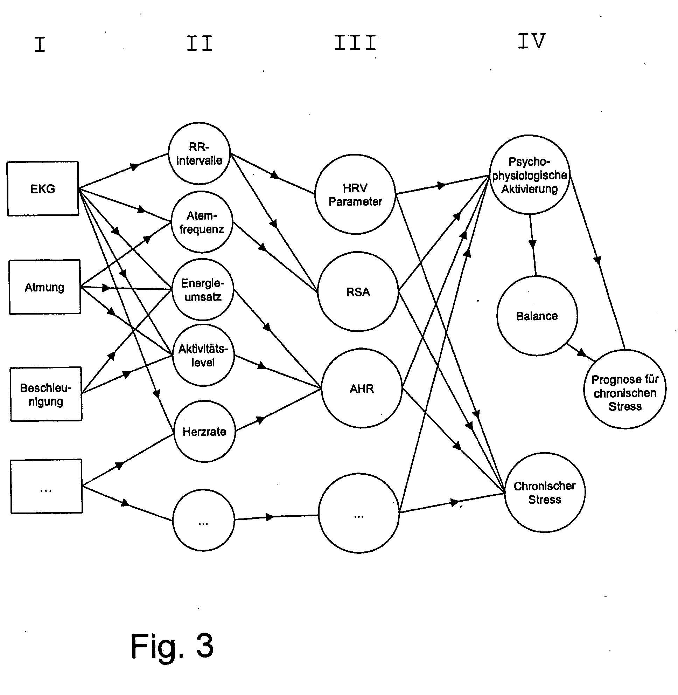

Die Fig. 1 zeigt in schematischer Darstellung die Komponenten der erfindungsgemäßen Vorrichtung. Eine Sensoreinrichtung 1 ist mittels Signalverbindungen 2, die drahtlos oder Drahtverbindungen sein können, mit mindestens einer Signalverarbeitungseinrichtung 3 verbunden. Die Sensoreinrichtung 1 ist in der bevorzugten Ausführungsform als Brustgurt ausgebildet. Es dient als direktes Messinstrument bestimmter Vitalparameter des Benutzers 4 (Fig. 2), wie z.B. dem Herzschlag oder der Atmung. Die gemessenen Daten werden in der Sensoreinrichtung 1 intern gespeichert und mit Hilfe des erfindungsgemäßen Verfahrens des mehrstufigen Algorithmus, welcher weiter unten anhand Fig. 3 näher erläutert wird, ausgewertet. Je nach Ergebnis der Ausgabeparameter wird eine aktive Rückmeldung an den Benutzer 4 ausgelöst.

Anhand mindestens einer Signalverarbeitungseinrichtung 3 in der bevorzugten Ausführungsform als Smartphone 3.1, PC 3.2 und/oder Uhr 3.3 realisiert, können die Ausgabeparameter und weitere Funktionalitäten des Sensorsystems 1 visualisiert, gespeichert und weiterverarbeitet werden. Der Benutzer 4 kann das Sensorsystem 1 mit Hilfe der Signalverarbeitungseinrichtung 3 nicht nur auslesen, sondern auch ferngesteuert bedienen, um persönliche Daten, eine zeitliche Auslöseverzögerung der aktiven Rückmeldung., Zusatzinformationen des Sensorsystems 1, ein Zeitprofil für die psychophysiologische Aktivierung und die Schwellwerte der Ober- bzw. Untergrenze der psychophysiologische Aktivierung einzustellen oder zu ändern. Die Uhr 3.3 weist im Vergleich zum Smartphone 3.1 oder dem PC 3.2 eine reduzierte Funktionalität auf, indem in der Uhr 3.3 nur einfache Bedienungselemente ausgebildet sind.

Verbunden ist das Sensorsystem 1 mit einer der Signalverarbeitungseinrichtungen 3 über eine Signalverbindung 2. Diese ist in bevorzugter Ausbildung zwischen der Sensoreinrichtung 1 und dem Smartphone 3.1 und/oder der Uhr 3.3 als eine drahtlose Signalverbindung, wie z.B. Bluetooth, realisiert. Die Signalverbindung 2 der Sensoreinrichtung 1 zum PC 3.2 kann neben Bluetooth und/oder WLAN wahlweise auch über eine drahtverbundene Signalverbindung, z.B. USB, ausgebildet sein. Signalverbindungen zwischen der Signalverarbeitungseinrichtung 3 und einer Webanwendung 3.4 im Internet, ermöglichen Datenaustausch zu elektronischen Tagebüchern, anderen Benutzern, einer Datenbank und/oder geben Zugriff zu Kontextinformationen aus dem Internet, wie z.B. aktuelle Wetterdaten, die direkt auf die Sensoreinrichtung 1 über eine der Signalverarbeitungseinrichtungen 3 übertragen werden können. Zusätzlich kann von der Sensoreinrichtung 1 auch eine direkte, drahtlose Signalverbindung 2 zu einer Webanwendung 3.4 aufgebaut werden.

In Fig. 2 sind die Komponenten des Sensorsystems 1 in Bezug auf den Benutzer 4, in Fig. 2 als Strichmännchen gezeichnet, in einem schematischen Diagramm dargestellt. Die Komponenten der Sensoreinrichtung sind neben mindestens einem Sensor 1.1, eine Signalisierungseinheit 1.2, eine Signalverarbeitung 1.3, die gefolgt von einem Speicher 1.4 über eine Telemetrie 1.5 mit den Signalverarbeitungseinrichtung 3 verbunden ist.

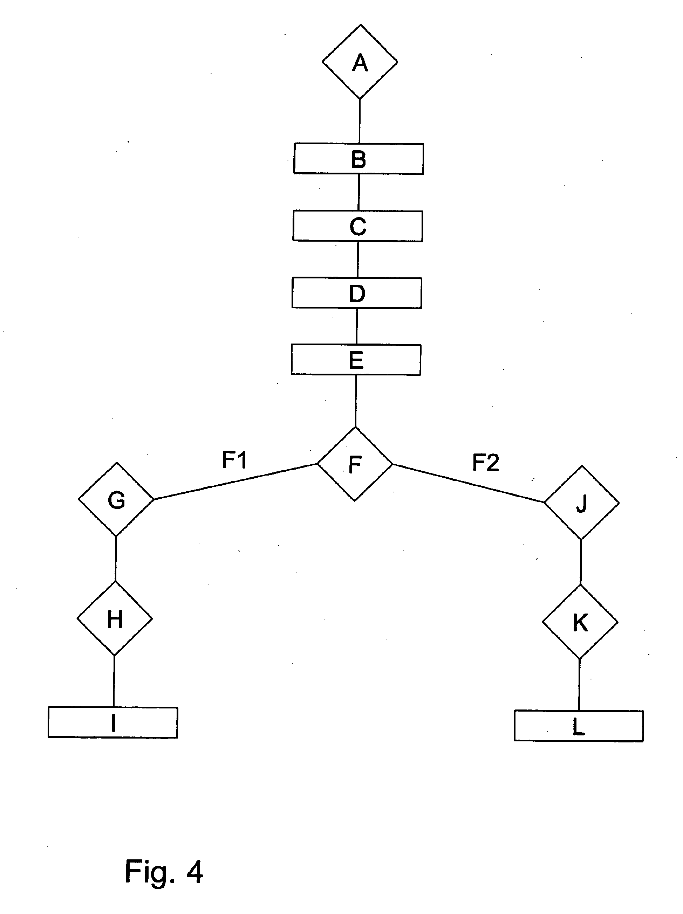

Der Sensor 1.1 nimmt Daten des Benutzers 4 durch Messung der Vitalparameter, z.B. Atmung, Beschleunigung oder das EKG des Benutzers 4 auf, wobei für jeden Vitalparameter mindestens ein Sensor 1.1 vorgesehen ist, und übergibt die Daten weiter an die Signalverarbeitung 1.3, die den erfindungsgemäßen mehrstufigen Algorithmus enthält, welcher weiter unten in Fig. 4 näher erläutert ist. Eine Signalisierungseinheit 1.2 dient als Signalgeber für den Benutzer 4 und gibt, je nach den errechneten Ausgabewerten aus der Signalverarbeitung 1.3, eine aktive Rückmeldung an den Benutzer 4. Die bevorzugte Ausführungsform ist eine taktile Situationsunterbrechung in Form eines Vibrationsgebers, der den Benutzer 4 durch Vibration informiert.

Ein Benutzer selbst hat zudem die Möglichkeit die Sensoreinrichtung 1 direkt durch einfache Berührung, einmaliges oder mehrmaliges Antippen des Geräts oder mit Hilfe einer Signalverarbeitungseinrichtung 3 zu bedienen und in diesem Zuge auch sogenannte "Marker" zu setzen. Diese sprechen bestimmte Stresssituationen oder bestimmte Bedienelemente der Sensoreinrichtung an, z.B. eine Aktivierung einer gewissen Verzögerungszeit oder der taktilen Situationsunterbrechung.

Die berechneten Ausgabewerte aus der Signalverarbeitung 1.3 werden in einem Speicher 1.4 intern im Sensorsystem 1 gespeichert. Mittels der Telemetrie 1.5 wird weiterhin eine Datenübertragung über die Signalverbindungen 2 zu mindestens einer der Signalverarbeitungseinrichtung 3 zur weiteren Auswertung vorgenommen, die in Fig. 2 zur besseren Unterscheidung und zur Kennzeichnung, dass diese nicht in der Ausführungsform des Sensorsystems 1 direkt integriert ist, gestrichelt gezeichnet ist. Im Gegenzug ermöglicht die Telemetrie 1.5 ebenfalls die umgekehrte Variante, dass Daten von der Signalverarbeitungseinrichtung 3 in die Signalverarbeitung 1.3 übertragen werden, um der Signalverarbeitung 1.3 spezielle Kontextinformationen oder veränderte Voreinstellungen zur Verfügung zu stellen.

In Fig. 3 ist ein Ausführungsbeispiel des mehrstufigen erfindungsgemäßen Verfahrens der Signalverarbeitung 1.3 dargestellt. In aufeinanderfolgenden Schritten ausgehend von gemessenen Sensordaten I werden zunächst bestimmte Merkmalsdaten II extrahiert, daraus definierte Parameterdaten III berechnet werden, welche entscheidend in die Ausgabeparameter IV eingehen. Dabei kann sowohl die Extraktion der Merkmalsdaten II, die Berechnung der Parameterdaten III, als auch die Berechnung der Ausgabewerte IV mehrstufig ausgelegt sein, wobei in dieser konkreten Ausführungsform mindestens zwei Berechnungsstufen ihre Anwendung finden.

In einem ersten Schritt werden mit Hilfe der Sensoren 1.1 des Sensorsystems 1 verschiedene Vitalparameter des Benutzers 4 in Form von Sensordaten I gemessen, die im Ausführungsbeispiel die Parameter Atmung, Körperbeschleunigung und eine Messung eines Elektrokardiogramms (EKG) beinhalten. Sodann werden in einem nächsten Schritt die bevorzugten Merkmalsdaten II aus den Sensordaten I extrahiert und gespeichert. Diese erste Berechnungsstufe dient dazu im Ausführungsbeispiel im Speziellen aus der Messung des EKG die RR-Abstände, die Atemfrequenz, den Energieumsatz, das Aktivitätslevel und die Herzrate aus den zuvor gemessenen Sensordaten I zu erhalten, wobei gleiche Merkmalsparameter II aus verschiedenen Sensordaten I extrahiert werden können, z.B. der Energieumsatz wird aus den Sensordaten I des EKG, der Atmung und der Beschleunigung extrahiert; dies verdeutlicht, dass eine äußerst genaue Bestimmung der Merkmalsparameter II möglich ist. Diese Merkmalsparameter II werden im darauffolgenden dazu benötigt, um in einer zweiten Berechnungsstufe weitere Parameterdaten III, wie die Herzratenvariabilität (HRV), die respiratorische Sinusarrhythmie (RSA) und additional heart rate (AHR) zu berechnen und für weitere Auswertungen zu speichern. Die Parameterdaten III werden hiernach in einer dritten Berechnungsstufe weiterverwendet, um durch eine vorerst letzte Berechnung die tatsächlichen Ausgabeparameter IV in Form der psychophysiologischen Aktivierung und der Balance der verschiedenen Aktivierungsbereiche des Benutzers 4 zu kalkulieren. Sodann werden aus den Parameterdaten III direkt die Werte der psychophysiologischen Aktivierung und des chronischen Stress des Benutzers 4 berechnet und aus der psychophysiologischen Aktivierung die Balance der verschiedenen Aktivierungsbereiche, sprich die Balance zwischen Über- und Unteraktivierung, bestimmt. Weiterhin wird aus Balance der verschiedenen Aktivierungsbereiche und der psychophysiologischen Aktivierung des Benutzers 4 eine Prognose für den zu erwartenden chronischen Stress in der Zukunft abgeschätzt.

Zusätzlich zu den bereits implementierten Vitalparametern und den daraus berechneten Zwischen- und Ausgabeparametern ist es möglich, dass der Algorithmus durch weitere Parameter und Berechnungsstufen ergänzt und erweitert wird.

Der Ablauf des erfindungsgemäßen Verfahrens mit Messung der Vitalparameter, Signalverarbeitung und eventueller Rückmeldung an den Benutzer ist in einem Ablaufdiagramm in Fig. 4 dargestellt. Im Rahmen einer konkreten Ausführungsform des erfindungsgemäßen Verfahrens ergeben sich im Einzelnen die folgenden Schritte:

Zunächst findet in Schritt A eine Abfrage statt, ob die Sensoreinrichtung 1, in der bevorzugten Ausführungsform mit dem Brustgurt, durch den Benutzer 4 angelegt wurde. Bei einer Bejahung der Abfrage schaltet sich das Sensorsystem 1 ein und beginnt in Schritt B die Erfassung der Sensordaten I, wobei neben den bereits genannten menschlichen Vitalitätscharakteristika, wie der Herztätigkeit, der Atmung und der Beschleunigung, ebenfalls weitere Daten, wie Körpertemperatur, Lage des Benutzers 4 etc., durch entsprechende Sensoren 1.1 erfasst werden können.

Schritt C beschreibt die Extraktion und Speicherung der Merkmalsdaten II aus den Sensordaten I, wobei aus einigen Sensordaten in der Kombination verschiedenste Merkmale berechnet werden können, wie sich z.B. der Energieumsatz aus den extrahierten Sensordaten der Beschleunigung, der Atmung und dem EKG zusammensetzt (siehe hierzu Fig. 3). Sodann beginnt in einem nächsten Schritt D die zweite Berechnungsstufe des erfindungsgemäßen mehrstufigen Algorithmus, indem aus den Merkmalsdaten die Parameterdaten III, die die wesentlichen Daten für die Berechnung der psychophysiologischen Aktivierung bereitstellen, berechnet und ebenfalls gespeichert werden. Hieraus werden in Schritt E in der dritten Berechnungsstufe die entsprechenden Ausgabeparameter IV für die psychophysiologische Aktivierung berechnet, gespeichert und ausgegeben.

Sodann erfolgt in Schritt F eine Abfrage, ob der vorgegebene Schwellwert der Obergrenze für die psychophysiologische Aktivierung (F1) überschritten wurde oder der vorgegebene Schwellwert der Untergrenze für die psychophysiologische Aktivierung unterschritten wurde (F2). Erfolgt Abfragemöglichkeit F1, sprich die Überschreitung des Schwellwertes der Obergrenze der psychophysiologischen Aktivierung, erfolgt mittels Schritt G eine Abfrage, ob ein Unterbrechungsmodus durch den Benutzer 4 aktiviert wurde und verfolgt bei positiver Rückmeldung Schritt H, in welchem abermals eine Abfrage auftritt, wobei geprüft wird, ob eine voreingestellte Verzögerungszeit eingehalten wurde, die bei Bejahung mittels Schritt I eine aktive Unterbrechung der Stresssituation durch ein Vibrationssignal zur Folge hat.

Sollte in Schritt I der Schwellwert der Untergrenze für die psychophysiologische Aktivierung unterschritten werden (F2), folgt der Ablauf der weiteren Geschehnisse der Schritte J, K und L. Dabei wird in Abfrage J abgefragt, ob der Aktivierungsmodus für eine Aktivierung des Benutzers 4 aktiviert ist. Bei einer Bejahung des vorherigen Schrittes J wird im folgenden Schritt K wiederum geprüft, ob die Verzögerungszeit eingehalten wurde und bei entsprechendem Ergebnis die aktive Rückmeldung in Schritt L für eine Aktivierung des Benutzers 4 durch ein Vibrationssignal an den Benutzer 4 gegeben.

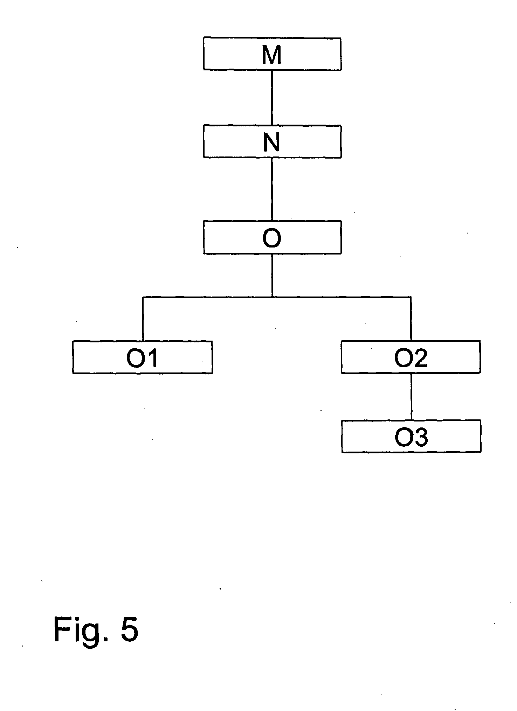

In Fig. 5 wird die Kalibrierung der erfindungsgemäßen Vorrichtung und des erfindungsgemäßen Verfahrens in einem schematisch skizzierten Ablaufdiagramm dargestellt, beginnend mit der Installation der benötigten Software über die Erstellung eines Benutzerprofils zu der Kalibrierung der Signalverarbeitung 1.3 für den erfindungsgemäßen mehrstufigen Algorithmus. Ein erster Schritt M bezeichnet die nötige Installation der Bedienungssoftware durch den Benutzer 4 in einer Signalverarbeitungseinrichtung 3, wobei diese in einer bevorzugten Ausführungsform ein PC 3.2 ist. Sodann legt der Benutzer 4 in einem nächsten Schritt N in der Bedienungssoftware ein Benutzerprofil an, welches die wichtigsten persönlichen Daten wie Größe, Alter, Gewicht, Geschlecht, Trainingszustand und normaler Ruhepuls des Benutzers 4 enthält. In Schritt O wird das Sensorsystem 1 als Brustgurt in der bevorzugten Ausgestaltung angelegt und für eine bestimmte Zeitspanne, z.B. eine Woche, getragen, um in dieser Zeitspanne die wichtigsten Parameter aufzunehmen, die als Basiswerte für die Signalbearbeitung 1.3 benötigt werden, wobei die eigentliche Kalibrierung der Signalbearbeitung 1.3 in die folgenden Schritte 01 - 03 aufgeteilt ist. In Schritt O1 werden verschiedene Funktionalitäten eingestellt, die auch während des Betriebes der erfindungsgemäßen Vorrichtung noch geändert werden können. Zum Einen können die Schwellwerte der Ober- und Untergrenze der psychophysiologischen Aktivierung nach Wunsch angepasst werden, Zeitprofile für aktive bzw. Ruhephasen eingestellt und die taktile Rückmeldung an- oder ausgeschaltet werden.

Während Schritt 02 werden innerhalb der Kalibrierungszeitspanne die Ruhe- bzw. Schlafphasen bestimmt, wobei die Bestimmung der Belastungsgrenzen beispielsweise durch die Durchführung eines normierten Stresstests erfolgt. Hiernach werden für die entsprechenden Parameterdaten Mittelwerte innerhalb einer solchen Ruhephase ermittelt, welche sodann als sogenannte Grundlinie für die jeweiligen Parameterdaten bzw. Merkmalsdaten dienen.

Die Werte der Kalibrierung stehen dann in einer Datenbank in einer Signalverarbeitungseinrichtung 3 und intern im Sensorsystem 1 gespeichert der Signalverarbeitung 1.3 zur Verfügung. In einer bevorzugten Ausführungsform läuft die Kalibrierung des Sensorsystems 1 in teil- oder vollautomatischer Weise ab, so dass die Bedienung der Sensoreinrichtung 1 so einfach wie möglich ist.

In einer bevorzugten Ausführungsform wird das erfindungsgemäße Verfahren des mehrstufigen Algorithmus innerhalb der erfindungsgemäßen Vorrichtung des Sensorsystems in der bevorzugten Ausführungsform als Brustgurt mit integriertem Sensor- und Auswertesystem zur Berechnung und Speicherung der psychophysiologischen Aktivierung, der Balance verschiedenen Aktivierungsbereiche bzw. der Ermittlung des chronischen Stress und der aktiven Rückmeldung mittels einer taktilen Situationsunterbrechung des Benutzers verwendet.

### Bezugszeichenliste

- 1: Sensoreinrichtung
- 1.1: Sensoren
- 1.2: Signalisierungseinheit
- 1.3: Signalverarbeitung
- 1.4: Speicher
- 1.5: Telemetrie
- 2: Signalverbindung
- 3: Signalverarbeitungseinrichtung,
- 3.1: Smartphone
- 3.2: PC
- 3.3: Uhr
- 3.4: Webanwendung
- 4: Benutzer
- I: Sensordaten
- II: Merkmalsparameter
- III: Parameterdaten
- IV: Ausgabeparameter
- A-L: Schritte des Ablaufdiagramms des erfindungsgemäßen Verfahrens
- M-O3: Schritte des Ablaufdiagramms der Kalibrierung

## Patentansprüche

1. System zum Stresstraining eines Benutzers mit einer Sensoreinrichtung (1), einer Signalverarbeitungseinrichtung (3) und mindestens einer Signalisierungseinheit (1.2), **dadurch gekennzeichnet, dass** die Signalverarbeitungseinrichtung (3) zur Bestimmung eines Werts für den Stress des Benutzers aus mindestens einem mit der psychophysiologischen Aktivierung des Benutzers (4) korrelierten Parameter ausgebildet ist und dass die Signalisierungseinheit (1.2) zur Aufgabe einer Rückmeldung an den Benutzer bei Über- bzw. Unterschreiten von definierten Schwellwerte der Aktivierung ausgebildet ist.

2. System nach Anspruch 1, **dadurch gekennzeichnet, dass** die Sensoreinrichtung (1) in einer am Körper tragbaren Ausgestaltung integriert ist, insbesondere als Brustgurt, Gürtel, Top oder Armbanduhr.

3. System nach Anspruch 1 oder 2, **dadurch gekennzeichnet, dass** die Sensoreinrichtung (1) automatisch kontextbezogen an- und ausschaltbar ist, insbesondere durch An- oder Ablegen in Form von Schließen des Brustgurtes, durch Körperwärme, bei An- bzw. Abwesenheit von Vitalparametern, durch Überwachung der Potentialdifferenz zweier Elektroden, durch Anschalten bei Bewegung und Abschalten bei vollständiger Ruhe.

4. System nach einem der Ansprüche 1 bis 3, **dadurch gekennzeichnet, dass** die Sensoren (1.1) der Sensoreinrichtung (1) mindestens als EKG-, EMG-, EDA-, Atmungs-, Puls-, Aktivitäts-, Bewegungs-, Lage-, Temperatur- und/oder Feuchtigkeitssensor ausgebildet sind.

5. System nach einem der Ansprüche 1 bis 4, **dadurch gekennzeichnet, dass** mindestens ein Sensor (1.1) der Sensoreinrichtung drahtlos mit der Signalverarbeitungseinrichtung (3) verbunden ist.

6. System nach einem der Ansprüche 1 bis 5, **dadurch gekennzeichnet, dass** mindestens ein externer Sensor als Temperatur-, Helligkeits- und/oder Lärmsensor ausgebildet und drahtlos mit der Signalverarbeitungseinrichtung (3) verbunden ist.

7. System nach einem der Ansprüche 1 bis 6, **dadurch gekennzeichnet, dass** die Signalverarbeitungseinrichtungen (3) als Smartphone (3.1), PC (3.2) und/oder Uhr (3.3) ausgestaltet sind.

8. System nach einem der Ansprüche 2 bis 7, **dadurch gekennzeichnet, dass** mindestens eine der Signalverarbeitungseinrichtungen (3) zumindest zeitweise drahtlos oder drahtverbunden über das Internet mit einer Webanwendung (3.4) verbunden ist, womit die Sensoreinrichtung (1) selbst zumindest zeitweise direkt über eine drahtlose Signalverbindung (2) mit dem Internet verbindbar ist und damit Kontextinformationen aus externen Datenquellen beziehbar sind.

9. System nach einem der Ansprüche 1 bis 8, **dadurch gekennzeichnet, dass** die Signalisierungseinheit (1.2), welche eine Rückmeldung an den Benutzer gibt, taktil als Vibrationsalarm und/oder als akustische und/oder optische Signalisierungseinheit ausgebildet ist.

10. System nach einem der Ansprüche 1 bis 9, **dadurch gekennzeichnet, dass** die Sensoreinrichtung (1) eine Eingabeeinrichtung (z.B. Taster) aufweist, mittels dessen Messdaten Markierungen für Ereignisse zuordenbar sind.

11. System nach Anspruch 10, **dadurch gekennzeichnet, dass** die Eingabeeinrichtung Eingaben mittels eines Beschleunigungssensor durch Antippen des Systems zulässt.

12. System nach einem der Ansprüche 1 bis 11, **dadurch gekennzeichnet, dass** Eingaben des Benutzers, insbesondere Markierungen für Ereignisse, über die Signalverarbeitungseinrichtung (3) oder das Internet erfassbar sind.

13. System nach einem der Ansprüche 1 bis 12, **dadurch gekennzeichnet, dass** die Sensoreinrichtung (1) zur Bestimmung mindestens eines Wertes für chronischen Stress und die Balance verschiedener Aktivierungsbereiche eines Benutzers ausgebildet ist.

14. System nach einem der Ansprüche 1 bis 13, **gekennzeichnet durch** einen internen Datenspeicher der Sensoreinrichtung (1) zum Speichern erfasster Sensordaten (I) und/oder daraus berechneten weiteren Merkmalsparametern (II), Parameterdaten (III) und/oder Ausgabeparametern (IV).

15. System nach Anspruch 14, **dadurch gekennzeichnet, dass** das Sensorsystem (1) mit Hilfe einer Signalverbindung (2) die gespeicherten Daten, ebenso aktuelle Sensordaten (I), Merkmalsparameter (II), Parameterdaten (III) und/oder Ausgabeparameter (IV) in Echtzeit, bestimmten Zeitintervallen und/oder bei Verfügbarkeit der Signalverbindung (2) an die Signalverarbeitungseinrichtung (3) überträgt.

16. System nach Anspruch 1, **dadurch gekennzeichnet, dass** die Sensoreinrichtung (1) konfigurierbar ist, indem insbesondere persönliche Daten, z.B. Größe, Alter, Geschlecht, Gewicht, Trainingszustand und/oder normaler Ruhepuls, ein Zeitprofil der gewünschten Zeitabschnitte für Aktivierung bzw. Situationsunterbrechung einer Stresssituation und persönliche Aktivierungswerte bzw. Auslöseverzögerungen einstellbar sind.

17. System nach Anspruch 16, **dadurch gekennzeichnet, dass** das Zeitprofil der gewünschten Zeitabschnitte für Aktivierung bzw. Situationsunterbrechung einer Stresssituation aus einem elektronischen Terminkalender erzeugbar ist.

18. System nach Anspruch 15 oder 16, **dadurch gekennzeichnet, dass** die Optionen Aktivierung und Situationsunterbrechung einer Stresssituation manuell aktivierbar bzw. deaktivierbar sind.

19. System nach einem der Ansprüche 1 bis 18, **dadurch gekennzeichnet, dass** die Sensoreinrichtung (1) individuell kalibrierbar ist, indem insbesondere einerseits Kalibrierungswerte aufgrund von Eigenschaften des Benutzers (4) aus einer Datenbank ermittelt werden, andererseits während einer bestimmten Tragedauer das Verhalten des Benutzers analysiert, d.h. explizit Ruhe- bzw. Schlafphasen erkannt werden, wobei für die entsprechenden Merkmalsparameter (II) und Parameterdaten (III) Mittelwerte innerhalb der Ruhephase ermittelt werden, die sodann als eine Grundlinie für die Merkmalsparameter (II) und Parameterdaten (III) dienen.

20. System nach Anspruch 19, **dadurch gekennzeichnet, dass** die Kalibrierung teilweise oder vollständig automatisch definierbar ist.

21. System nach einem der Ansprüche 1 bis 20, **dadurch gekennzeichnet, dass** gemessene Sensordaten (I) mittels simultaner Datenverarbeitung Merkmalsparameter (II) extrahierbar sind, daraus Parameterdaten (III) erzeugbar sind, die mit mindestens einem Ausgabeparameter (14) korrelieren.

22. System nach Anspruch 21, **dadurch gekennzeichnet, dass** sowohl die Extraktion der Merkmalsparameter (II), die Berechnung der Parameterdaten (III), als auch die Berechnung der Ausgabeparameter (IV) mehrstufig ausgelegt ist.

23. System nach Anspruch 21 oder 22, **dadurch gekennzeichnet, dass** die extrahierten Merkmalsparameter (II) mindestens einer der folgenden Parameter entspricht: Herzrate, Atemfrequenz, Energieumsatz, Aktivitätslevel, RR-Abstände, Aktivitätsklasse, Muskeltonus und phasische und tonische Werte der elektrodermalen Aktivität (EDA).

24. System nach einem der Ansprüche 1 bis 23, **dadurch gekennzeichnet, dass** die Parameterdaten (III) durch mindestens einen mit der psychophysiologischen Aktivierung korrelierten Parameter Herzratenvariabilität (HRV), respiratorische Sinusarrhythmie (RSA) und/oder additional heart rate (AHR) bestimmt sind.

25. System nach einem der Ansprüche 21 bis 24, **dadurch gekennzeichnet, dass** aus den Parameterdaten (III) die psychophysiologische Aktivierung und der chronische Stress berechnet wird, wobei aus der psychophysiologische Aktivierung die Balance zwischen Über- bzw. Unteraktivierung bestimmt und aus psychophysiologische Aktivierung und Balance eine Prognose des zu erwartenden chronischen Stress schätzbar ist.

26. System nach einem der Ansprüche 1 bis 25, **dadurch gekennzeichnet, dass** die Signalqualität automatisch bewertet und/oder Störungen verringert und derart die Qualität der Ausgabewerte verbessert wird.

27. Verfahren zum Stresstraining eines Benutzers, **dadurch gekennzeichnet, dass** die Signalverarbeitungseinrichtung (3) zur Bestimmung eines Werts für den Stress des Benutzers (4) aus mindestens einem mit der psychophysiologischen Aktivierung des Benutzers (4) korrelierten Parameter ausgebildet ist und, dass die Signalisierungseinheit zur Aufgabe einer Rückmeldung an den Benutzer bei Über- bzw. Unterschreiten von definierten Schwellwerte der Aktivierung ausgebildet ist.

28. Verfahren nach Anspruch 27, **dadurch gekennzeichnet, dass** aus gemessenen Sensordaten (1) Merkmalsparameter (II) extrahiert werden, hieraus Parameterdaten (III) erzeugt werden, aufgrund derer mindestens ein Ausgabeparameter (IV) bestimmt wird.

29. Verfahren nach Anspruch 28, **dadurch gekennzeichnet, dass** die Extraktion der Merkmalsparameter (II) und/oder die Berechnung der Parameterdaten (III) und/oder die Berechnung der Ausgabeparameter (IV) mehrstufig erfolgt.

30. Verfahren nach Anspruch 27 oder 28, **dadurch gekennzeichnet, dass** in die Bestimmung eines Merkmalsparameters (II) mindestens zwei Sensordaten (I), in die Bestimmung von Parameterdaten (III) mindesten zwei Merkmalsparameter und/oder in die Bestimmung von Ausgabeparametern mindestens zwei Parameterdaten eingehen.

31. Verfahren nach einem der Ansprüche 27 bis 30, **dadurch gekennzeichnet, dass** als Sensordaten Herzfrequenz (EGK), Atmung und/oder Beschleunigung des Benutzers gemessen werden.

32. Verfahren nach einem der Ansprüche 27 bis 31, **dadurch gekennzeichnet, dass** aus den Sensordaten (I) mindestens einen der Merkmalsparameter (II) Herzrate, Atemfrequenz, Energieumsatz, Aktivitätslevel, RR-Abstände, Aktivitätsklassen, Muskeltonus und/oder phasische und tonische Werte der elektrodermalen Aktivität (EDA) extrahiert werden.

33. Verfahren nach einem der Ansprüche 27 bis 32, **dadurch gekennzeichnet, dass** als Parameterdaten (III) mindestens einen mit der psychophysiologischen Aktivierung korrelierender Parameter aus Herzratenvariabilität (HRV), respiratorische Sinusarrhythmie (RSA), additional heart rate (AHR), Pulstransitzeit (PTT) und/oder Bewegungsunruhe bestimmt werden.

34. Verfahren nach einem der Ansprüche 27 bis 33, **dadurch gekennzeichnet, dass** aus den Parameterdaten (III) die psychophysiologische Aktivierung und der chronische Stress berechnet wird, wobei aus der psychophysiologische Aktivierung die Balance zwischen Über- bzw. Unteraktivierung bestimmt und aus psychophysiologische Aktivierung und Balance eine Prognose des zu erwartenden chronischen Stress schätzbar ist.

35. Verfahren nach einem der Ansprüche 27 bis 34, **dadurch gekennzeichnet, dass** die Signalqualität automatisch bewertet und/oder Störungen verringert und derart die Qualität der Ausgabewerte verbessert wird.
